# EUROPEAN PATENT APPLICATION

(11) **EP 3 804 689 A1**
(43) Date of publication of application: **14.04.2021**
(21) Application number: 20212747.8
(22) Date of filing: 09.12.2020
(51) Int. Cl.: A61K 8/19, A61K 8/22, A61K 8/34, A61K 8/49, A61P 31/04, A61Q 11/00, A61Q 11/02

(54) **GARGLING AGENT COMPRISISNG A SET OF COUPLED SUBSTANCES, METHOD OF USE AND CONTROL OF THE CASEUM**

(30) Priority: 09.10.2019 BR 102019021197
(71) Applicant: Jabbur Junior, Luiz, MG 30310-403 Belo Horizonte (BR)
(72) Inventor: Jabbur Junior, Luiz, MG 30310-403 Belo Horizonte (BR)
(74) Representative: v. Bezold & Partner Patentanwälte - PartG mbB

(57) **Abstract**

The present invention provides a gargling agent to control the caseum, gargling, a method of adjusting efficiency and power, according to the user, to caseum control, which comprises an application method, with agitation of the container, turbulence, ways of gargling, variations in relation to the procedures of use and applications in phases. The phases of the gargling agent to the caseum control can be used in different intensities or separated according to the caseum control to be obtained. It is evident that there is a lack of a gargling agent in the market, aimed at acting in the cavities in the oral region, more specifically close to the throat, which aims at controlling the caseum manifestation simultaneously with the control of the physiological environment conducive to its causes, bacteria, viruses and waste. One of the important features of the gargling agent to control the caseum, gargling, presented in the present invention is the intentional fact of having a coupled action of the substances in the formulation that generate the caseum control effects, in the throat cavities where they occur.

## Description

### BACKGROUND

The substance, yellowish or whitish, pasty, of varying sizes, formed in the oral environment, called caseum (FONSECA et al., 2012), or Latin cas 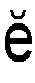 um or *casěus* (REZENDE; BIANCHET, 2014), or tonsil stones, or tonsilloliths, hereinafter referred to as caseum, is an oral manifestation, more specifically in cavities, in the throat region. Its appearance occurs through the action of bacteria in food residues (TSUNEISHI et al., 2006), dead cells, or hyperactive salivary glands and as a consequence a mass of unpleasant odor accumulates in this area. The caseum constitutes a social and health problem since it can cause much discomfort to individuals. The most common way to control it is by means of mechanical action, in an appropriate manner, for expulsion of which, as described in US patent number 2016361078 (A1). However, the efficiency of this method depends on the physical features of accessibility to the location, so it is not guaranteed that this control will be efficient in all cases, and it cannot be performed in any environment or at any time. In addition, the mechanical action of removing the caseum can cause a natural vomiting reflex.

The agents, in the form of a chemical solution, which can control the caseum, available on the market, demonstrate that they act indirectly on the bacteria that form it. CONCEIÇÃO et al. (2008) developed a solution, and classified it as a mouthwash, the formulation of which indicates only the antibacterial action and its control has been done by measuring the emission of sulfur compounds, such as hydrogen sulphide, originating from bacteria present in the caseum and consequently the intensity of its occurrence, and in the occurrence of long-term caseum, focusing on bad breath.

Also in the concept of mouthwash, or mouth rinse, the BR patent number 112015002969-8 B1 deals with a mouthwash, with antibacterial actions and that acts indirectly on the products of the actions of bacteria as well. The direct attack on the surface of the caseum structure is not evident in any of the cases available in the literature and these cases are linked only to antibacterial action.

One of the important features of the gargling agent for caseum control, gargling, presented in the present invention, is the intentional fact of having a coupled action of the substances in the formulation that generates the caseum control effects in the throat cavities where they occur, that is, the set of agents, substances, of individual action is already known in the literature (MUSSATTO; ROBERTO, 2002; CHANDEL et al., 2017; WESTPHALEN et al., 2016), such as cetylpyridinium chloride (National Center for Biotechnology Inf. Pub Chem Database. Cetylpyridinium chloride, CID=31239, <https://pubchem.ncbi.nlm.nih.gov/compound/Cetylpyridinium-chloride> accessed on Oct. 07, 2019) and in BR patent number 112015002969-8 B1, when submitted to the formulation method and use of this invention, they comprise a quantitative minimization action of the caseum, with superior efficiency, and focused on the caseum surface, to the use of the substances used for creation of the gargling agent of this invention.

It is evident that there is a lack of a gargling agent in the market, aimed at acting in the cavities in the oral region, more specifically close to the throat, which aims at controlling the caseum manifestation simultaneously with the control of the physiological environment conducive to its causes, bacteria, viruses and waste. It is noteworthy that caseum, as it acts as a site of proliferation of viruses and bacteria, can cause bad breath, known as halitosis, viral and bacterial tonsillitis and/or tongue plaque ratifying the importance of controlling thereof for the maintenance of health and well-being.

### SUMMARY

The present invention provides a gargling agent to control the caseum, gargling, a method of adjusting efficiency and power, according to the user, to control the caseum, which comprises an application method, with agitation of the container, turbulence, ways of gargling, variations in relation to the procedures of use and applications in phases. The phases of the gargling agent to control the caseum can be used in different intensities or separated according to the caseum control to be obtained.

The base formulation of phase 1, of the gargling agent, has the chlorine atom (CI) as a coupler, in the concept of chemical interaction of substances. The basic formulation, from where originate the percentage variations of the composition of the gargling agent, phase 1, is assembled with quaternary salt AB, the dissociation of which in aqueous medium generates cation A and anion B, sodium bicarbonate, the dissociation of which in aqueous medium generates sodium cation and bicarbonate anion, and sodium chloride (NaCI), the dissociation of which in aqueous medium generates the chloride anions and sodium cation, derived from the dissociations of salt AB and bicarbonate, according to the total chemical balance given by the interaction of the basic components. The basis of phase 1 is then composed of the coupled interaction expressed by AB plus sodium bicarbonate that generates cations A and sodium, and B anions, bicarbonate, and chloride.

The basic formulation of phase 2 is composed of a nanotechnological adsorbent material (FERREIRA; RANGEL, 2009), or particulate, such as activated carbon (AC), activated carbon and hydrogen peroxide (PEH). The CA, by the phenomenon of adsorption (FUKUMOTO; KURODA, 2019), which is explored in nanotechnology, adheres to the caseum and by the gargling action exposes more caseum to the action of nascent oxygen coming from the PEH that in turn can enter the structure of the caseum and induces the small parts of the caseum to be disaggregated, decreasing its size by erosion.

### BRIEF DESCRIPTION OF THE TECHNOLOGY

The method for the control of tonsil caseum, objective of the present invention, introduces the combined use of substances and gargling techniques to offer the functionality to quantitatively reduce the tonsil caseum manifestation.

The method for gargling with the gargling agent, compared to other methods of oral hygiene, physically interacts to disaggregate caseum particles and is still an efficient antibacterial agent, which reinforces the purpose of the present invention. The gargling is made according to the persistence of the caseum manifestation, which can be:
- agitation of the vial containing phase 1 of the gargling agent and immediate application in the form of gargling, agitation of the vial containing phase 2 of the gargling agent and immediate application in the form of gargling, once a day before brushing;
- agitation of the vial containing phase 1 of the gargling agent and immediate application in the form of gargling, agitation of the vial containing phase 2 of the gargling agent and immediate application in the form of gargling, once a day before brushing;
- agitation of the vial containing phase 1 of the gargling agent and immediate application in the form of gargling, agitation of the vial containing phase 2 of the gargling agent and immediate application in the form of gargling, once a day before brushing;
- agitation of the vial containing phase 1 of the gargling agent and immediate application in the form of gargling, agitation of the vial containing phase 2 of the gargling agent and immediate application in the form of gargling, after each meal.

The formulations of stages 1 and 2, for the execution of the method of use, can be presented in different forms, which can be:
- Solution in aqueous medium for phase 1, ready for use, added or not with flavoring, essential oils, dye and solution in aqueous medium, for phase 2, ready for use, added or not with flavoring, essential oils, coloring;
- Solution composed by mixing the solutions of phase 1 and 2 in a single container;
- powder preparation, of phases 1 and 2, for dilution in water at the place of use;
- concentrated liquid preparation, of phases 1 and 2, for dilution on demand;
- powder preparation, for dilution in a single dose. In all possibilities, additives for foam reduction and pH displacement, for higher or lower values, can be added.

### BRIEF DESCRIPTION OF THE DRAWINGS

In order to illustrate the description, reference is made to the attached drawings, wherein:
- Figure 1 shows the diagram of formulation phase 1.
- Figure 2 shows the complete formulation diagram, which comprises from phase 1e to phase 2.

### DETAILED DESCRIPTION

Hereinafter some examples are given and explored, which have been tested, description and basic embodiments, which are not intended to limit the invention as well as its form and method of application. These are examples that punctuate practical, simple, and explanatory formulations.

The present invention, which comprises a gargling agent designed to control the intensity at which the tonsil caseum is generated, decrease in the tonsil caseum existing before the use of the gargling agent, is composed by a basic formulation, with adjustable chemical substances concentrations in line with the method of using the gargling agent. The efficiency of the invention is centered on the correct combination of the formulation, method of use and observation of the effects for the optimal adjustment of this formulation/method combination.

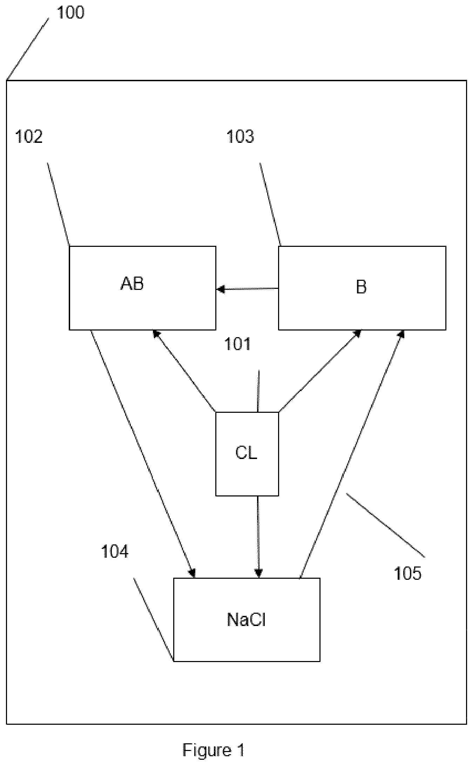

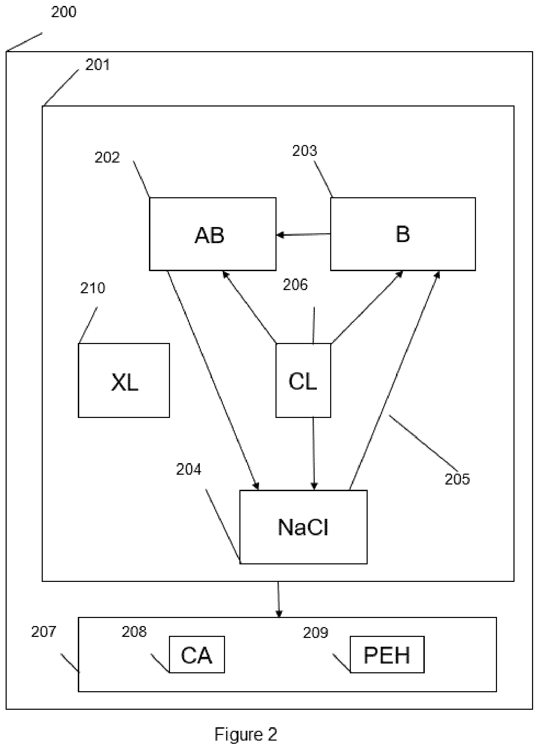

According to the drawing in Figure 1, the gargling agent provides a formulation, characterized as Phase 1 item (100), which prepares the oral environment for the actuation of the formulation characterized as Phase 2, shown in Figure 2, represented by block (207), where (100) and (207) can act individually or simultaneously in the same application and use (200).

The formulation of Phase 1 (100) is established by three substances coupled by an ion in common, CI, represented by the CL block (206). (206) comes from three substances, quaternary salt AB (202), sodium bicarbonate B (203) and sodium chloride (204). As each individual has a different feature in the caseum formation, regarding the formation intensity, salivary conditions, frequency of occurrence, the concentrations of (202), (203) and (204), optimal, can be a population average (individuals with tonsil caseum) or adjusted in order to personalize the gargling agent.

The formulation of Phase 2 (207) is established by two substances represented by the CA block (208), which deals with active carbon and the PEH block (209), which deals with hydrogen peroxide, the optimal concentrations of which can be a population average (individuals with tonsil caseum) or adjusted in order to personalize the gargling agent.

Phases 1 and 2 can act separately temporally, first (100) and later (207) or simultaneously (200) in the same application, mixed, depending on the ideal concentrations and the way that the tonsil caseum manifests itself in the individual, or in the chronic conditions and continuous use. Hereinafter formulations of variants of the gargling agent are introduced, which are not limited to the examples. For example, the invention introduces a range of formulations, oriented according to diagram and items organized and shown in Figure 2 (200), contemplating (201) and (207), with the following configuration and composition:
- cetylpiridinium chloride (202) at a concentration of 0.01 to 1%;
- xylitol (210) at a concentration of 1.0 to 20%;
- sodium bicarbonate (203) at a concentration of 0.5 to 10%;
- sodium chloride (204) at a concentration of 0.5% or indirect equivalent of (102) and (103)%;
- activated carbon (208) at a concentration of 0.001 to 1.0%;
- hydrogen peroxide (209) at a concentration of 0 to 3.0%;
- water in an amount to complete the volume.

In another variant, the present invention introduces the previous gargling agent wherein the addition of an antifoam occurs. For example, the addition of 1; 0% of polydimethylsiloxane.

In another variant, the present invention introduces a gargling agent comprising a cetylpiridinium chloride part, a sodium bicarbonate part, a xylitol part, an activated carbon part, a hydrogen peroxide part and a water part. For example, the invention introduces a formulation, oriented according to diagram and items organized and shown in Figure 2 (200), contemplating (201) and (207), with the following configuration and composition:
- cetylpiridinium chloride (202) at a concentration of 0.06%;
- xylitol (210) at a concentration of 10.0%;
- sodium bicarbonate (203) at a concentration of 0.05%;
- sodium chloride (204) at a concentration of 0.5%;
- activated carbon (208) at a concentration of 1.0%;
- hydrogen peroxide (209) at a concentration of 3.0%;
- water in an amount to complete the volume.

In another variant, the present invention introduces a gargling agent comprising a cetylpiridinium chloride part, a sodium bicarbonate part, a xylitol part, an activated carbon part, a hydrogen peroxide part and a water part. For example, the invention introduces a formulation, oriented according to diagram and items organized and shown in Figure 2 (200), contemplating (201) and (207), with the following configuration and composition:
- cetylpiridinium chloride (202) at a concentration of 0.05%;
- xylitol (210) at a concentration of 5.0%;
- sodium bicarbonate (203) at a concentration of 0.5%;
- sodium chloride (204) at a concentration of 0.5%;
- activated carbon (208) at a concentration of 1.0%;
- hydrogen peroxide (209) at a concentration of 3.0%;
- water in an amount to complete the volume.

In another variant, the present invention introduces the previous gargling agent wherein the addition of an antifoam occurs. For example, the addition of 1; 0% of polydimethylsiloxane.

In another variant, the present invention introduces a gargling agent comprising a cetylpiridinium chloride part, a sodium bicarbonate part, a xylitol part, an activated carbon part, a hydrogen peroxide part and a water part. For example, the invention introduces a formulation, oriented according to diagram and items organized and shown in Figure 2 (200), contemplating (201) and (207), with the following configuration and composition:
- cetylpiridinium chloride (202) at a concentration of 0.06%;
- xylitol (210) at a concentration of 10.0%;
- sodium bicarbonate (203) at a concentration of 0.05%;
- sodium chloride (204) at a concentration of 0.5%;
- activated carbon (208) at a concentration of 1.0%;
- hydrogen peroxide (209) at a concentration of 3.0%;
- water in an amount to complete the volume.

In another variant, the present invention presents the previous gargling agent wherein the addition of an antifoam occurs. For example, the addition of 1; 5% polydimethylsiloxane.

In another variant, the present invention introduces a gargling agent comprising a cetylpiridinium chloride part, a sodium bicarbonate part, a xylitol part, an activated carbon part, a hydrogen peroxide part and a water part. For example, the invention presents a formulation, oriented according to diagram and items organized and shown in Figure 2 (200), contemplating (201) and (207), with the following configuration and composition:
- cetylpiridinium chloride (202) at a concentration of 0.08%;
- xylitol (210) at a concentration of 10.0%;
- sodium bicarbonate (203) at a concentration of 5.0%;
- sodium chloride (204) at a concentration of 0.5%;
- activated carbon (208) at a concentration of 1.0%;
- hydrogen peroxide (209) at a concentration of 3.0%;
- water in an amount to complete the volume.

In another variant, the present invention presents the previous gargling agent wherein the addition of an antifoam occurs. For example, the addition of 1; 0% polydimethylsiloxane.

In another variant, the present invention presents a gargling agent comprising a cetylpiridinium chloride part, a sodium bicarbonate part, a xylitol part, an activated carbon part, a hydrogen peroxide part without water addition, oriented for dilution at the time of use, as a variation of the method of use. For example, the invention presents a formulation, oriented according to diagram and items organized and shown in Figure 2 (200), contemplating (201) and (207), with the following configuration and composition:
- cetylpiridinium chloride (202) at a concentration of 0.06%;
- xylitol (210) at a concentration of 10.0%;
- sodium bicarbonate (203) at a concentration of 1.0%;
- sodium chloride (204) at a concentration of 0.5%;
- activated carbon (208) at a concentration of 1.0%;
- hydrogen peroxide (209) at a concentration of 3.0%.

In another variant, the present invention presents the previous gargling agent wherein the addition of an antifoam occurs. For example, the addition of 1; 0% polydimethylsiloxane.

In another variant, the present invention introduces a gargling agent comprising a cetylpiridinium chloride part, a sodium bicarbonate part, a xylitol part, and a water part. With (204) created by the CI and Na ions from (202) and (203), respectively. For example, the invention presents a formulation, oriented according to diagram and items organized and shown in Figure 2 (200), contemplating (201), with the following configuration and composition:
- cetylpiridinium chloride (202) at a concentration of 0.06%;
- xylitol (210) at a concentration of 10.0%;
- sodium bicarbonate (203) at a concentration of 1.0%;
- water in a quantity to complete the volume.

In another variant, the present invention presents the previous gargling agent wherein the addition of an antifoam occurs. For example, the addition of 1; 0% polydimethylsiloxane.

### REFERENCES

CHANDEL, S .; KHAN, M .; SINGH, N.; AGRAWAL, A.; KHARE, V. National Journal of Maxillofacial Surgery, v. 8, p. 106, 07 2017.
CONCEIÇÃO, M. D. d.; MAROCCHIO, L. S.; TARZIA, O. Rev. Bras. scielo, v. 74, p. 61-67, 02 2008. ISSN 0034-7299.
FERREIRA, H. S.; RANGEL, M.d. C. Nanotecnologia: aspectos gerais e potencial de aplicação em catálise. Química Nova, scielo, v. 32, p. 1860 - 1870, 002009. ISSN 0100-4042.
FONSECA, F.; FALCATO, J. A.; ANDERSON, F.; ALMEIDA, J. N. de; TOJINHA, M. Dicionário Médico. Rua do Vale Formoso, 37 1959-006 Lisbon: [s.n.], 2012.
FUKUMOTO, A. A. F.; KURODA, E. K. Engenharia Sanitaria e Ambiental, scielo, v. 24, p. 295 - 304, 04 2019. ISSN 1413-4152.
MUSSATTO, S. I.; ROBERTO, I. C. Xilitol. Rev. Bras. Cienc. Farm, scielo, v. 38, p. 401 - 413, 12 2002. ISSN 1516-9332.
REZENDE, A. M. de; BIANCHET, S. B. Dicionário do latim essencial. [S.I.: s.n.], 2014.12/12.
TSUNEISHI, M.; YAMAMOTO, T.; KOKEGUCHI, S.; TAMAKI, N.; FUKUI, K.; WATANABE, T. Composition of the bacterial flora in tonsilloliths. Microbes and Infection, Elsevier Masson SAS, v. 8, n. 9-10, p. 2384-2389, 8 2006. ISSN 1286-4579. Quoted on page 1.

## Claims

1. Gargling agent comprising a set of coupled substances, method of use and control of the caseum, **characterized by** comprising a gargling agent in an amount necessary to attack the caseum, decrease the intensity of the caseum manifestation, wherein the gargling agent is at least one pyridinium salt compound as a cation and chlorine as an anion, together with sodium bicarbonate, together with sodium chloride, together with xylitol, together with hydrogen peroxide, together with a nanoporous, nanotechnological or particulate adsorbent, wherein the pyridinium salt comprises cetylpyridinium chloride at a concentration of 0.01 to 0.1%, wherein sodium bicarbonate is present at a concentration of 0.5 to 10.0%, wherein the adsorbent is activated carbon at a concentration of 0.001 to 1%, wherein the presence of chloride is given by the presence of chlorine and sodium ions derived from sodium bicarbonate and cetylpyridinium chloride, wherein xylitol is present at a concentration of 1 to 20%, wherein the hydrogen peroxide varies in a range of 0 to 3%, as it directly attacks the caseum and interferes with the structure therein.

2. Gargling agent comprising a set of coupled substances, method of use and control of the caseum, according to claim 1, **characterized by** further comprising an amount of a foaming controller, a dye, a preservative, a pH control by means of a chemical buffer solution.

3. Gargling agent comprising a set of coupled substances, method of use and control of the caseum, **characterized by** comprising a gargling agent in an amount necessary to attack the caseum, decrease the intensity of the caseum manifestation, wherein the gargling agent is at least one of nanoporous, nanotechnological or particulate adsorbent compound.

4. Gargling agent comprising a set of coupled substances, method of use and control of the caseum, **characterized by** further comprising an amount of a foaming controller, a dye, a preservative, a pH control by means of a chemical buffer solution.

5. Gargling agent comprising a set of coupled substances, method of use and control of the caseum, **characterized by** comprising a gargling agent in an amount necessary to attack the caseum, decrease the intensity of the caseum manifestation, wherein the gargling agent it is at least one pyridinium salt compound as a cation and chlorine as an anion, together with sodium bicarbonate, together with sodium chloride, together with xylitol, together with hydrogen peroxide, together with a nanoporous, nanotechnological or particulate adsorbent, wherein the pyridinium salt comprises cetylpyridinium chloride at a concentration of 0.06%, wherein sodium bicarbonate is present at a concentration of 0.5%, wherein the adsorbent is activated carbon at a concentration of 0.025%, wherein the presence of chloride is confirmed by the presence of chlorine and sodium ions from sodium bicarbonate and cetylpyridinium chloride, wherein xylitol is present at a concentration of 10%, wherein the hydrogen peroxide varies in a range of 1%, as it directly attacks the caseum and interferes with the structure therein, as it is antiseptic.

6. Gargling agent comprising a set of coupled substances, method of use and control of the caseum, according to claim 5, **characterized by** further comprising an amount of a foaming controller, a dye, a preservative, a pH control by means of a chemical buffer solution.

7. Gargling agent comprising a set of coupled substances, method of use and control of the caseum, **characterized by** a gargling agent in an amount necessary to attack the caseum, decrease the intensity of the caseum manifestation, wherein the gargling agent is at least one pyridinium salt compound as a cation and chlorine as an anion, together with sodium bicarbonate, together with sodium chloride, together with xylitol, wherein the pyridinium salt comprises cetylpyridinium chloride at a concentration of 0.01 to 0.1%, wherein sodium bicarbonate is present at a concentration of 0.5 to 10.0%, wherein the presence of chloride is confirmed by the presence of chlorine and sodium ions from sodium bicarbonate and cetylpyridinium chloride, wherein xylitol is present at a concentration of 1 to 20%, as it directly attacks the caseum and interferes with the structure therein.

8. Gargling agent comprising a set of coupled substances, method of use and control of the caseum, according to claim 7, **characterized by** further comprising an amount of a foaming controller, a dye, a preservative, a pH control by means of a chemical buffer solution.

9. Gargling agent comprising a set of coupled substances, method of use and control of the caseum, **characterized by** a gargling agent in an amount necessary to attack the caseum, decrease the intensity of the caseum manifestation, wherein the gargling agent is at least one pyridinium salt compound as a cation and chlorine as an anion, together with sodium bicarbonate, together with sodium chloride, together with xylitol, wherein the pyridinium salt comprises cetylpyridinium chloride at a concentration of 0.055%, wherein sodium bicarbonate is present at a concentration of 0.5%, wherein the presence of chloride is confirmed by the presence of chlorine and sodium ions from sodium bicarbonate and cetylpyridinium chloride, wherein xylitol is present at a concentration of 10%, as it directly attacks the caseum and interferes with the structure therein, as it is antiseptic.

10. Gargling agent comprising a set of coupled substances, method of use and control of the caseum, according to claim 9, **characterized by** further comprising an amount of a foaming controller, a dye, a preservative, a pH control by means of a chemical buffer solution.

11. Gargling agent comprising a set of coupled substances, method of use and control of the caseum, according to claim 1, **characterized by** further comprising an amount of a foaming controller, a dye, a preservative, a pH control by means of a chemical buffer solution, a method of use with continuous agitation and uses at different intervals and times of the day.
